# EUROPEAN PATENT APPLICATION

(11) **EP 1 897 873 A1**
(43) Date of publication of application: **12.03.2008**
(21) Application number: 07013953.0
(22) Date of filing: 17.07.2007
(51) Int. Cl.: C07D 311/24

(54) **A process for the preparation of nitrile compounds used as intermediates in the synthesis of cysteinyl leukotrienes antagonists**

(30) Priority: 09.08.2006 IT MI20061606
(71) Applicant: Dipharma Francis S.r.l., 20021 Baranzate (MI) (IT)
(72) Inventor: Colombo, Lino, 27100 Pavia (IT); Allegrini, Pietro, 20097 San Donato Milanese (MI) (IT); Amorosi, Alessandra, 27100 Pavia (IT); Razzetti, Gabriele, 20099 Sesto San Giovanni (MI) (IT); Rossi, Roberto, 27100 Pavia (IT)
(74) Representative: Minoja, Fabrizio

(57) **Abstract**

A process for the preparation of a compound of formula (I) or a salt thereof 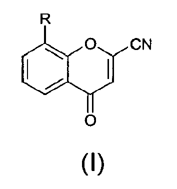 wherein
R is an amino, nitro or -NHCOR₁ group, wherein R₁ is C₁-C₆ alkyl or phenyl optionally substituted by a -O-(CH₂)ₙ-phenyl group, wherein n is an integer of 1 to 6; comprising reacting a compound of formula (II) or a salt thereof, both as the individual isomer and as an isomeric mixture, 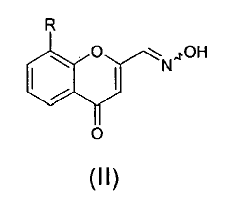 wherein R is as defined above; with a dehydrating agent, if necessary in the presence of an organic solvent, and optionally in the presence of a basic agent; and, if desired, converting a compound of formula (I) to another compound of formula (I) or a salt thereof.

## Description

### FIELD OF THE INVENTION

The present invention relates to a novel process for the preparation of nitrile compounds useful as intermediates in the synthesis of e.g., pranlukast and its structural analogues. Pranlukast, a selective competitive antagonist of cysteinyl leukotrienes C(4), LTD(4) and LTE(4), has the following formula 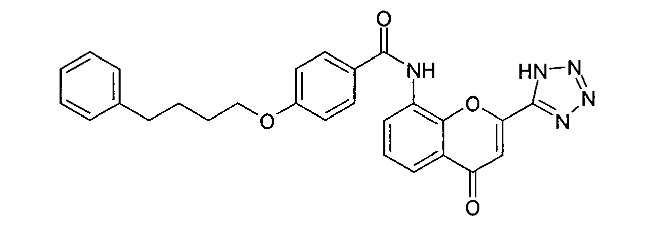 and is useful in the treatment and prophylaxis of bronchial asthma in both pediatric and adult patients.

### TECHNOLOGICAL BACKGROUND

A cyano-derivative compound of formula (I), as herein defined, is a key intermediate for the preparation of pranlukast according to the processes disclosed in EP 173516. The preparation of said cyano-derivative is carried out by dehydration of the corresponding amides. EP 634409 discloses that the dehydration reaction of said amide involves various drawbacks and problems, which can only be solved by effecting dehydration under specific conditions and in the presence of an excess of pyridine derivatives. There is therefore a need for an alternative synthesis of the intermediate of formula (I) through processes more suited to the industrial application.

### SUMMARY OF THE INVENTION

An alternative process has now been found for the preparation of nitrile compounds of formula (I), as herein defined, which overcomes the problems of the prior art and affords said compounds in a purity equal to or higher than 99.5%. The use of an intermediate with such a high purity allows to obtain pranlukast in a purity equal to or higher than 99.5%.

### DETAILED DISCLOSURE OF THE INVENTION

An object of the invention is a process for the preparation of a compound of formula (I), or a salt thereof 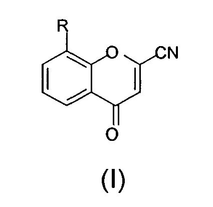 wherein R is an amino, nitro or -NHCOR₁ group, wherein R₁ is C₁-C₆ alkyl or phenyl optionally substituted by a -O-(CH₂)ₙ-phenyl group, wherein n is an integer of 1 to 6;
which process comprises the reaction of a compound of formula (II) or a salt thereof, both as the individual isomer and as an isomeric mixture, 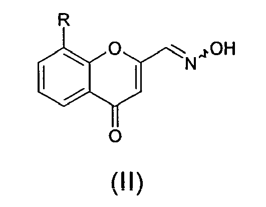 wherein R is as defined above;
with a dehydrating agent, if necessary in the presence of an organic solvent, and optionally in the presence of a basic agent;
and, if desired, the conversion of a compound of formula (I) to another compound of formula (I) or a salt thereof.

An isomer of a compound of formula (II) can be for example a geometric isomer thereof.

A salt of a compound of formula (I) is for example a salt with an acid, preferably a mineral acid, for example hydrochloric acid.

A C₁-C₆ alkyl group, which can be straight or branched, is for example a C₁-C₄ alkyl group, preferably methyl, ethyl, propyl, isopropyl, butyl or isobutyl, more preferably methyl or ethyl.

In a -(CH₂)ₙ- chain, which can be straight or branched, n is preferably an integer of 1 to 4, more preferably 4.

When R₁ is phenyl, this is preferably para-substituted.

A salt of a compound of formula (II) is for example a salt with an organic or inorganic base, typically a trialkylamine, in particular triethylamine.

A dehydrating agent is for example an agent selected from acetyl chloride, trichloroacetyl chloride, an organic acid anhydride, typically acetic anhydride, trifluoromethanesulfonic anhydride or trifluoroacetic anhydride, phenyl chloroformate, carbonyl bis-imidazole, trichloroacetonitrile, Vilsmeier reagent (chloromethylenedimethylimminium chloride), trichlorocyanuric acid, trichloroisocyanuric acid, phosphorous pentoxide, phosphorous trichloride, phosphorous tribromide, phosphoryl chloride, phenyl dichlorophosphate, diphenyl chlorophosphate, diphenyl phosphite, sulfur dichloride, sulfur monochloride, thionyl chloride, chlorosulfonyl isocyanate and phenyl sulphenyl chloride; preferably an organic acid anhydride, more preferably acetic anhydride.

An organic solvent can be for example an aprotic solvent, typically an ether, in particular tetrahydrofuran, dioxane or diethyl ether; a chlorinated solvent, e.g. dichloromethane, or an aliphatic hydrocarbon e.g. hexane, pentane or cyclohexane or an aromatic hydrocarbon, e.g. toluene; or an excess of dehydrating agent as defined above, particularly acetic anhydride. Hexane, toluene and acetic anhydride are preferred.

A basic agent is typically a tertiary amine, in particular a trialkylamine, e.g. triethylamine, tributylamine or ethyl diisopropylamine; or a heterocyclic amine such as pyridine, lutidine or collidine; preferably triethylamine.

The dehydration reaction can be carried out at a temperature approximately ranging from -10°C to the reflux temperature of the reaction mixture, preferably at a temperature approximately ranging from 80°C to the reflux temperature of the reaction mixture.

The stoichiometric ratio of the dehydrating agent to a compound (II), or a salt thereof, can approximately range from 1 to 20, preferably approximately from 1 to 2.

The stoichiometric ratio of the basic agent to a compound (II) can approximately range from 0.5 to 2, preferably approximately from 1 to 1.5.

The optional conversion of a compound of formula (I) to another compound of formula (I) or a salt thereof can be carried out according to known methods.

In addition, a compound of formula (I) or a salt thereof, can be converted to a compound of formula (IA) or a salt thereof 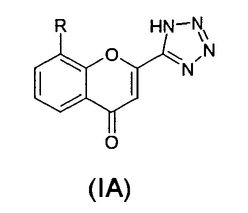 wherein R is as defined above, in particular a -NHCOR₁ group, wherein R₁ is phenyl para-substituted by -O-(CH₂)₄-phenyl, according to known methods, for instance as disclosed in EP 173516.

A salt of a compound of formula (IA) is for example a pharmaceutically acceptable salt as exemplified in EP 173516.

A compound of formula (I) or a salt thereof, thus obtained, has purity equal to or higher than 99.0%, in particular equal to or higher than 99.5%. The use of an intermediate with such high purity allows to obtain a compound of formula (IA) or a salt thereof, in particular pranlukast, with a purity equal to or higher than 99.0%, in particular equal to or higher than 99.5%.

The compounds of formula (II) and the salts thereof, both as the individual isomers and as an isomeric mixture, are novel compounds and are an object of the invention.

Preferred examples of compounds of formula (II) are:
o 8-nitro-4-oxo-4*H*-chromene-2-carboxyaldehyde oxime;
o 8-amino-4-oxo-4*H*-chromene-2-carboxyaldehyde oxime;
o *N*-(2-hydroxyiminomethyl-4-oxo-4*H*-chromen-8-yl)-4-(4-phenylbutoxy)-benzamide
and the salts thereof, both as the individual isomers and as an isomeric mixture.

A compound of formula (II) or a salt thereof, as defined above, both as the individual isomer and as an isomeric mixture, can be obtained by a process comprising:
a) the reaction of a compound of formula (III) 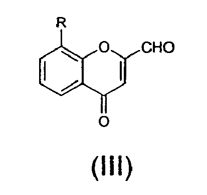 wherein R is as defined above,
   with hydroxylamine or a salt thereof, in an organic solvent, optionally in the presence of a basic agent; or
b) the reaction of a compound of formula (IV) 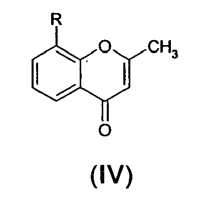 wherein R is nitro, with a compound of formula (V)

   R₂-O-N=O (V)

   wherein R₂ is C₁-C₆ alkyl, in the presence of a basic agent and, if necessary, in an organic solvent, to obtain a compound of formula (II) wherein R is nitro;
and, if desired, the separation of an isomeric mixture into the individual isomers and/or the conversion of a compound (II) to another compound (II) or a salt thereof.

A compound (II) or a salt thereof, thus obtained, has purity equal to or higher than 99.0%, in particular equal to or higher than 99.5%.

The reaction of a compound (III) with hydroxylamine can be carried out at a temperature approximately ranging from -10°C to the reflux temperature of the reaction mixture, preferably at a temperature approximately ranging from 0°C to 30°C.

Hydroxylamine can be used in an approximately 10 to 50% aqueous solution, preferably approximately 30 to 50%.

The stoichiometric ratio of hydroxylamine to a compound (III) can approximately range from 1.0 to 2.0, preferably approximately from 1.5 to 2.0.

A hydroxylamine salt can be a mineral acid salt, for example hydroxylamine hydrochloride, hydroxylamine sulfate or hydroxylamine phosphate; preferably hydroxylamine hydrochloride.

The reaction of a compound (III) with a hydroxylamine salt, in the presence of a basic agent, can be carried out at a temperature approximately ranging from -10°C to the reflux temperature of the reaction mixture, preferably at a temperature approximately ranging from 0°C to 30°C.

The stoichiometric ratio of the hydroxylamine salt to a compound of formula (III) can range approximately from 1.0 to 2.0; preferably approximately from 1.5 to 2.0.

A basic agent can be a tertiary amine, typically a trialkylamine, e.g. triethylamine, tributylamine or ethyl diisopropylamine; or a heterocyclic amine e.g. pyridine, 2,6-lutidine, 3,5-lutidine or collidine; or an alkali or alkaline-earth carbonate or hydrogen carbonate, e.g. sodium or potassium carbonate or calcium bicarbonate or carbonate; preferably triethylamine.

An organic solvent can be an aprotic solvent, typically an ether, e.g. tetrahydrofuran, dioxane or diethyl ether; or an alcohol, e.g. methanol, ethanol, isopropanol or n-butanol; preferably tetrahydrofuran.

In a compound of formula (V) a C₁-C₆ alkyl group, which can be straight or branched, can be for example a C₁-C₅ alkyl group, preferably ethyl, propyl, butyl, isobutyl, amyl or isoamyl; more preferably isoamyl.

The reaction between a compound of formula (IV) and a compound of formula (V) can be carried out in the presence of a basic agent, which can be for example an alkali or alkaline-earth hydride such as sodium hydride, potassium hydride or calcium hydride; an alkali alkoxide e. g. sodium or potassium methoxide, sodium or potassium ethoxide, sodium or potassium tert-butoxide; preferably sodium or potassium ethoxide.

An organic solvent can be an ether, e.g. ethyl ether, tetrahydrofuran or dioxane; a dipolar aprotic solvent, e.g. dimethylformamide or dimethyl sulfoxide; preferably an ether solvent, more preferably tetrahydrofuran.

The reaction between a compound of formula (IV) and a compound of formula (V) can be carried out at a temperature approximately ranging from -10°C to the reflux temperature of the reaction mixture, preferably at a temperature approximately ranging from 0°C to 30°C.

A compound of formula (II) can be converted to another compound of formula (II) or a salt thereof with known methods. Analogously, the separation of its isomeric mixture into its individual isomers can be carried out with known methods.

A compound of formula (III) is novel and can be prepared by hydrolysis of a compound of formula (VI), both as the individual isomer and as an isomeric mixture, 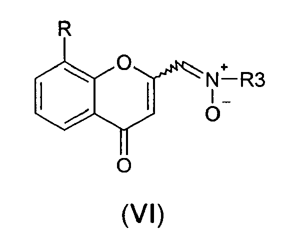 wherein R is as defined above and R₃ is aryl, optionally substituted with a -N(R₄R₅) group wherein each of R₄ and R₅ is independently C₁-C₆ alkyl;
in the presence of water, optionally in the presence of an acid, if necessary in the presence of an organic solvent, optionally with heating.

An isomer of a compound of formula (VI) can be for example a geometric isomer thereof.

An aryl group is for example phenyl or naphthyl, preferably phenyl.

A C₁-C₆ alkyl group, which can be straight or branched, is for example a C₁-C₄ alkyl group, preferably methyl, ethyl, propyl, isopropyl, butyl or isobutyl, more preferably methyl or ethyl.

Preferred examples of compounds of formula (III) are:
o *N*-(2-formyl-4-oxo-4*H*-chromen-8-yl)-4-(4-phenylbutoxy)-benzamide;
o 2-formyl-8-amino-4-oxo-4*H*-chromene; and
o 2-formyl-8-nitro-4-oxo-4*H*-chromene.

An acid can be for example a mineral acid e.g. sulfuric acid, hydrochloric acid, hydrobromic acid or an organic acid e.g. a sulfonic acid, e.g. para-toluenesulfonic acid, methanesulfonic acid, acetic acid, formic acid, oxalic acid; preferably a sulfonic acid, more preferably para-toluenesulfonic acid.

An organic solvent can be an ether solvent, e.g. tetrahydrofuran or dioxane; a dipolar aprotic solvent, e.g. acetone, dimethylformamide or dimethylacetamide; or an alkanol, e.g. methanol, ethanol, propanol, isopropanol; preferably an ether, more preferably tetrahydrofuran.

The hydrolysis reaction can be carried out at a temperature approximately ranging from -10°C to the reflux temperature of the reaction mixture, preferably at a temperature approximately ranging from +10°C to room temperature.

The water volume percentage in the organic solvent can approximately range from 1 to 10%.

The stoichiometric ratio of the acid to a compound of formula (VI) can approximately range from 0 to 0.2, preferably approximately 0.1.

The compounds of formula (IV) and (V) are known or can be obtained according to known methods.

A compound of formula (VI), both as the individual isomer and as an isomeric mixture, is novel and can be obtained by reaction between a compound of formula (IV), as defined above, and a compound of formula (VII) 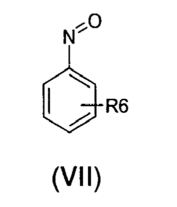 wherein R₆ is for example hydrogen, hydroxy, C₁-C₆ alkyl or a -N(R₄R₅) group as defined above, preferably a dialkylamino group, more preferably 4-dimethylamino, in the presence of one or more basic agents, optionally in an organic solvent.

Preferred examples of compounds of formula (VI), both as the individual isomers and as an isomeric mixture, are:
o *N*-(2-((4-dimethylamino)-phenyl-1-nitronyl)-4-oxo-4*H*-chromen-8-yl)-4-(4-phenylbutoxy)-benzamide;
o 2-((4-dimethylamino)-phenyl-1-nitronyl)-8-nitro-4-oxo-4*H*-chromene; and
o 2-((4-dimethylamino)-phenyl-1-nitronyl)-8-amino-4-oxo-4*H*-chromene.

The reaction between a compound of formula (IV) and a compound of formula (VII) is typically carried out in the presence of 1, 2 or 3 of the above defined basic agents; preferably 1 or 2 of them.

An organic solvent can be an ether, e.g. ethyl ether, tetrahydrofuran or dioxane; a dipolar aprotic solvent e.g. dimethylformamide or dimethyl sulfoxide; preferably an ether solvent, more preferably tetrahydrofuran.

The reaction between a compound of formula (VI) and a compound of formula (VII) can be carried out at a temperature approximately ranging from -10°C to the reflux temperature of the reaction mixture, preferably at a temperature approximately ranging from 0°C to 30°C. The compounds of formula (VII) are known or can be obtained with known methods.

The following examples illustrate the invention.

### EXAMPLE 1: N-(2-((4-Dimethylamino)-phenyl-1-nitronyl)-4-oxo-4H-chromen-8-yl)-4-(4-phenylbutoxy)-benzamide (VI)

*n*-2-Methyl-4-oxo-4*H*-chromen-8-yl)-4-(4-phenylbutoxy)-benzamide (IV) (200 mg; 0.468 mmols) is dissolved in 5 ml of anhydrous THF at 25°C and added with K₂CO₃ (323.4 mg; 2.34 mmols), then with potassium tert-butoxide (52.52 mg; 0.468 mmols) and, after 10 minutes, with solid *p*-dimethyl-nitrosoaniline (210.8 mg, 1.404 mmols). After 4 hours, TLC shows the disappearance of the starting compound. The reaction is quenched by addition of a NH₄Cl saturated solution and brine, then is extracted with dichloromethane, dried and the two isomers are purified by flash chromatography on silica, affording a 58% overall yield.

Following the same procedure, the following compounds are obtained:
o 2-((4-dimethylamino)-phenyl-1-nitronyl)-8-nitro-4-oxo-4*H*-chromene; and
o 2-((4-dimethylamino)-phenyl-1-nitronyl)-8-amino-4-oxo-4*H*-chromene.

### EXAMPLE 2: N-(2-Formyl-4-oxo-4H-chromen-8-yl)-4-(4-phenylbutoxy)-benzamide (III)

Compound (VI) (500 mg; 0.89 mmols), prepared according to Example 1, is dissolved in THF (5.0 ml) at room temperature, then added with *p*-toluenesulfonic acid (17 mg, 0.089 mmols) and water (0.5 ml). The reaction is carried out at room temperature under magnetic stirring. After 3 hours the reaction is complete by TLC. A sodium bicarbonate saturated solution is added and the mixture is extracted with ethyl acetate. The organic phase is washed with 1 M HCl, citric acid, finally water, then dried and the residue is purified by flash chromatography on silica to obtain a 60% yield in aldehyde (III).

Following the same procedure, the following compounds are obtained:
o 2-formyl-8-amino-4-oxo-4*H*-chromene; and
o 2-formyl-8-nitro-4-oxo-4*H*-chromene.

### EXAMPLE 3: N-(2-(Hydroxyimino-methyl)-4-oxo-4H-chromen-8-yl)-4-(4-phenylbutoxy)-benzamide (II)

Aldehyde (III) (158 mg; 0.356 mmols), prepared according to Example 2, is suspended in ethanol (3.5 mL) and added with hydroxylamine hydrochloride (50 mg; 0.7 mmols) at room temperature and triethylamine (100 µL; 0.7 mmols) is dropped therein. After 24 hours, the mixture is evaporated to residue, which is taken up in dichloromethane, washed with diluted acid, dried and evaporated to dryness. The solid crude obtained in quantitative yield is directly subjected to the subsequent reaction.

Following the same procedure, starting from the appropriate intermediates, the following compounds are obtained:
o 8-nitro-4-oxo-4*H*-chromene-2-carboxyaldehyde oxime and
o 8-amino-4-oxo-4*H*-chromene-2-carboxyaldehyde oxime.

### EXAMPLE 4: N-(2-Cyano-4-oxo-4H-chromen-8-yl)-4-(4-phenylbutoxy)-benzamide (I)

Crude oxime (II) (456 mg, 1.0 mmols), obtained according to Example 3, is added with acetic anhydride (5.0 ml) and refluxed for an hour at 138-140°C. Acetic anhydride is then evaporated off under reduced pressure and the residue is purified by flash chromatography on silica, to obtain the desired chromone nitrile (I) in 80% yield and HPLC purity higher than 99%.

### EXAMPLE 5: 8-Nitro-4-oxo-4H-chromene-2-carboxyaldehyde oxime (II)

10 g (0.049 mols) of 2-methyl-8-nitro-chromen-4-one are dissolved in 50 ml of ethanol, the mixture is cooled to 0-5°C and 70 g of a 20% sodium ethoxide solution in ethanol are dropped therein. 15.2 g (0.147 mols) of freshly prepared butyl nitrite are subsequently added. The mixture is reacted for 2 hours (temperature spontaneously raises), then added with acetic acid to pH below 7 and concentrated to dryness. The resulting product is purified by flash chromatography. 9.8 g of 8-nitro-4-oxo-4*H*-chromene-2-carboxyaldehyde oxime (II) are obtained, with HPLC purity higher than 99%.

## Claims

1. A process for the preparation of a compound of formula (I) or a salt thereof 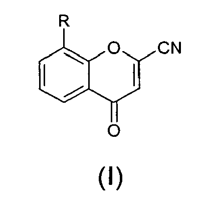 wherein
R is an amino, nitro or -NHCOR₁ group, wherein R₁ is C₁-C₆ alkyl or phenyl optionally substituted by a -O-(CH₂)ₙ-phenyl group, wherein n is an integer of 1 to 6;
which process comprises the reaction of a compound of formula (II) or a salt thereof, both as the individual isomer and as an isomeric mixture, 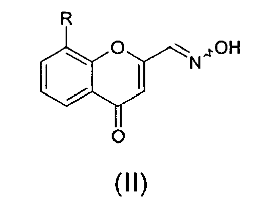 wherein R is as defined above;
with a dehydrating agent, if necessary in the presence of an organic solvent, and optionally in the presence of a basic agent; and, if desired, the conversion of a compound of formula (I) to another compound of formula (I) or a salt thereof.

2. The process as claimed in claim 1, wherein the dehydrating agent is selected from acetyl chloride, trichloroacetyl chloride, an organic acid anhydride, phenyl chloroformate, carbonyl bis-imidazole, trichloroacetonitrile, Vilsmeier reagent (chloromethylenedimethylimminium chloride), trichlorocyanuric acid, trichloroisocyanuric acid, phosphorous pentoxide, phosphorous trichloride, phosphorous tribromide, phosphoryl chloride, phenyl dichlorophosphate, diphenyl chlorophosphate, diphenyl phosphite, sulfur dichloride, sulfur monochloride, thionyl chloride, chlorosulfonyl isocyanate and phenyl sulphenyl chloride.

3. The process as claimed in claim 2, wherein the dehydrating agent is selected from acetic anhydride and trifluoroacetic anhydride.

4. The process as claimed in claim 1, wherein the organic solvent is selected from an ether, a chlorinated solvent, an aliphatic or aromatic hydrocarbon, or an excess of dehydrating agent as defined in claim 3.

5. The process as claimed in claim 1, wherein the basic agent is selected from a tertiary amine, a heterocyclic amine, an alkali or alkaline-earth carbonate or hydrogen carbonate.

6. The process as claimed in claim 1, wherein the stoichiometric ratio of the dehydrating agent to a compound (II) or a salt thereof, approximately ranges from 1 to 20.

7. The process as claimed in claim 1, wherein the stoichiometric ratio of the basic agent to a compound (II) or a salt thereof, approximately ranges from 0.5 to 2.

8. The process as claimed in claim 1, further comprising the conversion of a compound of formula (I) or a salt thereof, to a compound of formula (IA) or a salt thereof 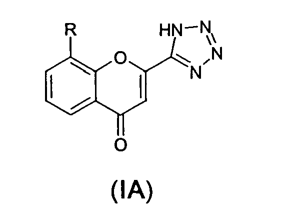 wherein R is as defined in claim 1.

9. A compound of formula (II) or a salt thereof, both as the individual isomer and as an isomeric mixture, 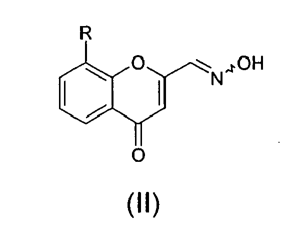 wherein R is an amino, nitro or -NHCOR₁ group, wherein R₁ is C₁-C₆ alkyl or phenyl optionally substituted by a -O-(CH₂)ₙ-phenyl group, wherein n is an integer of 1 to 6.

10. A compound as claimed in claim 9, selected from:
o 8-nitro-4-oxo-4*H*-chromene-2-carboxyaldehyde oxime;
o 8-amino-4-oxo-4*H*-chromene-2-carboxyaldehyde oxime; and
o *N*-(2-hydroxyiminomethyl-4-oxo-4*H*-chromen-8-yl)-4-(4-phenylbutoxy)-benzamide.

11. A compound of formula (III) or (VI) as individual isomer or isomeric mixture, 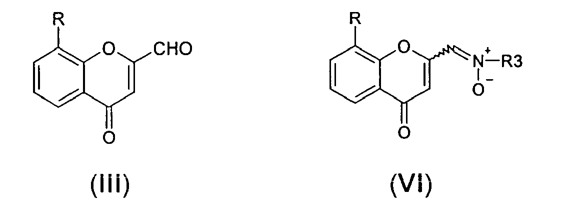 wherein R is an amino, nitro or -NHCOR₁ group, wherein R₁ is C₁-C₆ alkyl or phenyl optionally substituted by a -O-(CH₂)ₙ-phenyl, wherein n is an integer of 1 to 6; and R₃ is aryl, optionally substituted with a -N(R₄R₅) group wherein each of R₄ and R₅ is independently C₁-C₆ alkyl.

12. A compound as claimed in claim 11, which is selected from:
o *N*-(2-formyl-4-oxo-4*H*-chromen-8-yl)-4-(4-phenylbutoxy)-benzamide;
o 2-formyl-8-amino-4-oxo-4*H*-chromene;
o 2-formyl-8-nitro-4-oxo-4*H*-chromene;
o *N*-(2-((4-dimethylamino)-phenyl-1-nitronyl)-4-oxo-4*H*-chromen-8-yl)-4-(4-phenylbutoxy)-benzamide;
o 2-((4-dimethylamino)-phenyl-1-nitronyl)-8-nitro-4-oxo-4*H*-chromene; and
o 2-((4-dimethylamino)-phenyl-1-nitronyl)-8-amino-4-oxo-4*H*-chromene.
